# EUROPÄISCHE PATENTANMELDUNG

(11) **EP 1 384 706 A1**
(43) Veröffentlichungstag der Anmeldung: **28.01.2004**
(21) Anmeldenummer: 03015745.7
(22) Anmeldetag: 10.07.2003
(51) Int. Cl.: C07C 67/343, C07C 69/34, C07C 51/09, C07C 255/03

(54) **Verfahren zur Monoalkylierung C-H-acider Methylengruppen**

(30) Priorität: 24.07.2002 DE 10233507
(71) Anmelder: Degussa AG, 40474 Düsseldorf (DE)
(72) Erfinder: Bodmann, Kerstin, Dr., 45772 Marl (DE); Meyer, Oliver, Dr., 48151 Münster (DE); Kaufhold, Manfred, Dr., 45770 Marl (DE); Fieker, Jürgen, 45701 Herten (DE); Paulczynski, Renate, 44623 Herne (DE)

(57) **Zusammenfassung**

Das vorliegende Verfahren umfasst die Herstellung monoalkylierter C-H-acider methylengruppenhaltiger Verbindungen wie z.B. Malonester, Malonesternitrile etc. Diese lassen sich vorteilhaft in einem polaren aprotischen Lösungsmittel in Gegenwart von Kaliumcarbonat als Base gewinnen, wenn die Base in einem Verhältnis von >0.6:1 zur C-H-aciden methylengruppenhaltigen Verbindung eingesetzt wird.

## Beschreibung

Die vorliegende Erfindung betrifft ein Verfahren zur Alkylierung von C-H-aciden Verbindungen. Insbesondere beschäftigt sich das vorliegende Verfahren mit der selektiven Herstellung von Monoalkylierungsprodukten ausgehend von C-H-aciden methylengruppentragenden Substraten und Dihalogenalkane in einem nichtwässrigen System.

Die Alkylierung von C-H-aciden Verbindungen ist eine Standardreaktion der organischen Chemie. Schwierig ist dabei die Unterdrückung von Dialkylierungsreaktionen, da insbesondere bei stark C-H-aciden Verbindungen die Acidität des Monoalkylderivats oft noch ausreicht, um eine weitere Reaktion mit einem Elektrophil zu begünstigen. Die einfach und doppelt alkylierten Derivate sind meistens jedoch schlecht zu trennen, weshalb gerade im technischen Maßstab eine möglichst selektive Reaktion gefordert wird (Organikum, VEB Deutscher Verlag der Wissenschaften, Berlin, 1986, S. 517).

Die Alkylierung sehr C-H-acider Verbindungen wie Malonester oder Malonesternitrilen ist aus der Literatur bekannt. Häufig werden dabei Natriumethanolat oder Natriumhydrid als Base eingesetzt, um die deprotonierten Derivate zu erzeugen, welche in einer Substitutionsreaktion anschließend mit dem Elektrophil, z.B. einem Alkylhalogenid, abgefangen werden (DE3401913; EP69648). Es hat auch schon Versuche gegeben, bei denen Kaliumcarbonat als Base zur Alkylierung von Malonestern eingesetzt wurde (Synth. Commun. 1977, 7, 559-568; Zh. Org. Khim. 1983, 19, 474-480; DE4326917). Während man bei den ebengenannten Schriften jeweils die Dialkylierung von Malonesterderivaten mit 1,2-Dihalogenalkanen zur Bildung von Cyclopropylderivaten anstrebte, ist in der DE19752041 auch die Monoalkylierung von Malonestern offenbart. Dabei wird die Umsetzung bei einem Molverhältnis von Malonsäurederivat zu Alkylierungsmittel zu Kaliumcarbonat von 1:1,5-3,0:0,4-0,6 propagiert. Für die Dialkylierung wird vorgeschlagen eine Basenmenge von ≥1 im Verhältnis zur C-H-aciden Verbindung zu benutzen.

Aufgabe der vorliegenden Erfindung war die Angabe eines weiteren Verfahrens zur Herstellung von Monoalkylierungsprodukten von C-H-aciden Verbindungen, insbesondere von Malonesterderivaten. Besonderes Augenmerk wird dabei auf deren Gewinnung im technischen Maßstab unter ökonomisch wie ökologisch vorteilhaften Gesichtspunkten gelegt. Insbesondere sollte das vorliegende Verfahren bessere Raum/Zeitausbeuten an Monoalkylierungsprodukten liefern als die Verfahren des Standes der Technik, wobei die Bildung inter- oder intramolekularer dialkylierter Produkte möglichst unterdrückt werden soll. Ebenfalls Aufgabe der Erfindung war, ein Herstellungsverfahren anzugeben, dass ausgehend von dem eben genannten Verfahren es in einfacher Weise gestattet, ω-Halgencarbonsäuren bzw. - nitrile aus den entsprechenden Malonesterderivaten unter ebenfalls geeigneten technischen Bedingungen herzustellen.

Diese und weitere nicht näher genannte sich jedoch aus dem Stand der Technik ergebende Aufgaben werden durch ein Verfahren mit den Merkmalen des gegenständlichen Anspruchs 1 bzw. 10 gelöst. Abhängige Unteransprüche betreffen bevorzugte Ausführungsformen der erfindungsgemäßen Verfahren.

Dadurch, dass man in einem Verfahren zur Monalkylierung C-H-acider Methylengruppen durch Reaktion des methylengruppentragenden Substrats in einem polaren aprotischen Lösungsmittel mit Dihalogenalkanen, bei denen die beiden Halogenatome durch eine Kette von mindestens 3 C-Atomen getrennt sind, in Gegenwart von Alkali- oder Erdalkalicarbonaten und einem Phasentransferkatalysator unter stetiger Entfernung des sich bildenden Reaktionswassers die Alkali- oder Erdalkalicarbonate im Verhältnis zum methylengruppentragenden Substrat von >0.6:1 einsetzt, gelangt man in überraschender dafür aber nicht minder vorteilhaften Weise zur Lösung der gestellten erstgenannten Aufgabe. Mit dem gegenständlichen Verfahren ist es entgegen der Lehre des Standes der Technik auch möglich, mit bestimmten Dihalogenalkanen C-H-aciden Verbindungen in für den technischen Maßstab sehr guten Raum/Zeitausbeuten zu monoalkylieren, wobei die inter- oder intramolekularen Dialkylierungsreaktionen weitgehend vermieden werden. So reagiert 1,6-Dichlorhexan unter den erfindungsgemäßen Bedingungen mit Malonsäurediestern innerhalb von 3 h in knapp 80% Ausbeute zu den gewünschten Monoalkylierungsprodukten, ohne dass bei dem Baseüberschuss Kettenbildung durch Mehrfachalkylierung ein Problem ist. Als Dihalogenalkane können alle dem Fachmann für diesen Zweck in Frage kommende bishalogenierte Alkane herangezogen werden, sofern beide Halogenatome durch eine Kette von mindestens 3 C-Atomen getrennt sind. Besonders bevorzugt ist der Einsatz von primären oder sekundären Dihalogenalkanen. Vorzugsweise sind dies primären α,ω-Dihalogenalkanen der Formel X-(CH₂)ₙ-Y mit n = 4-14 wie z.B. 1,4-Dichlorbutan, 1,6-Dichlorhexan, 1, 8-Dichloroctan, 1,10- Dichlordecan, 1,6-Bibromhexan, 1,8-Dibromoctan. X und Y können dabei gleich oder verschieden Fluor, Chlor, Brom oder Jod sein. Insbesondere sind aus Umwelt- und Kostengründen die jeweiligen Chloride bevorzugt als Alkylhalogenide einzusetzen.

Bevorzugt ist der Einsatz von Alkali- oder Erdalkalicarbonaten im Verhältnis zum methylengruppentragenden Substrat von >1:1, mehr bevorzugt >1,1:1, >1,3:1 und äußerst bevorzugt von >1,4:1.
Eine Obergrenze für den Einsatz der Basenmenge ergibt sich für den Fachmann aus technischen und kommerziellen Gesichtspunkten heraus. Die Rührbarkeit der Suspension oder die Minimierung von Stoffeinsatzkosten z.B. stellen Randbedingungen dar, an denen sich der Fachmann orientieren wird. In diesem Bereich kann der Fachmann die Basenmenge für optimale Reaktionsbedingungen jedoch frei wählen. Ein Verhältnis von ca. 1,5:1 (Base:C-H-acider Verbindung) hat sich als sehr geeignet in diesem Zusammenhang herausgestellt.

In einer bevorzugten Ausführungsform gibt man den Phasentransferkatalysator kontinuierlich ab Beginn der Alkylierungsreaktion zur Reaktionsmischung zu. Zum einen lässt sich damit die exotherme und gasbildende Reaktion sehr gut kontrollieren, auf der anderen Seite beschleunigt dies die Reaktionsgeschwindigkeit noch weiter. Im Gegensatz dazu lehrt die DE19752041, dass die gewünschten Monoalkylierungsderivate dann besonders gut erhalten werden, wenn man den einzusetzenden Phasentransferkatalysator erst ab etwa 50%-80%igem Umsatz des eingesetzten Malonesters hinzufügt.
Optional kann sich nach dem Ende der Zugabe des Katalysators noch eine Nachreaktionszeit bis zu 2/3 - 1/4 der Gesamtreaktionszeit anschließen. Das Ende der Reaktion ist an der nachlassenden Gas und Wasserentwicklung abzulesen.

C-H-acide methylengruppentragende Moleküle sind dem Fachmann geläufig. Prinzipiell stellt sich das Problem der Dialkylierung vermehrt bei stark C-H-aciden Verbindungen, weshalb hier die vorliegende Erfindung besonders gut einsetzbar ist. Verbindungen mit stark C-H-aciden Methylengruppen sind insbesondere solche, welche in Nachbarstellung spezielle Gruppen tragen, die einen elektronenziehenden -I-Effekt auf besagte Methylengruppe ausüben. Dieser kann durch in gleiche Richtung wirkende mesomere Effekte verstärkt sein. Besonders hervorzuheben sind in diesem Zusammenhang 1,3-Dicarbonylverbindungen. Diese besitzen Carbonyl- bzw. Carboxylgruppen in direkter Nachbarschaft zur Methylengruppe und sorgen mit ihrem -Iund -M-Effekt für eine extreme C-H-Acidität dieser Verbindungsklasse. Besonders bevorzugt ist daher der Einsatz von Verbindungen wie Malonsäurediester - mit gleichen oder verschiedene Estergruppen, Cyanessigsäureestern, Acetoaceton, β-Oxocarbonsäureester. Ganz besonders bevorzugt ist der Einsatz von Malonsäurediestern bzw. Malonsäuredinitrilen oder Cyanessigsäureestern. Als Estergruppen in solchen Verbindungen dienen vorzugsweise primäre, sekundäre oder tertiäre (C₁-C₈) -Alkylgruppen.

Als Alkali- oder Erdalkalicarbonate stehen dem Fachmann prinzipiell alle für diesen Zweck in Frage kommenden Verbindungen zur Verfügung. Aus Kostengründen sind Natrium- bzw. Kaliumcarbonat zu bevorzugen. Ebenfalls vorteilhaft sind Lithium- bzw. Magnesium- und Calciumcarbonat. Ganz besonders bevorzugt ist der Einsatz von Kaliumcarbonat in diesem Zusammenhang. Das eingesetzte Carbonat hat vorzugsweise einen Feinkornanteil von 85% < 0,1 nm.

Ein essentielles Merkmal der vorliegenden Erfindung ist, dass sich während der Reaktion bildende Wasser aus der Reaktionsmischung zu entfernen. Hierfür können prinzipiell alle dem Fachmann für diesen Zweck in Frage kommenden Substrate benutzt werden. Vorteilhaft können Molsiebe, Orthoester oder azeotrope Schleppmittel eingesetzt werden. Im Hinblick auf die Auswahl geeigneter Schleppmittel ist der Fachmann frei. U.U. können auch die eingesetzten Dihalogenalkane selbst als Schleppmittel eingesetzt werden. Bevorzugt insbesondere beim Einsatz höher siedender Alkylhalogenide ist zur Entfernung des Reaktionswassers ein Schleppmittel ausgewählt aus der Gruppe der cyclischen Kohlenwasserstoffe oder aromatischen Kohlenwasserstoffe zu nehmen. Ganz besonders bevorzugt sind Cyclohexan, Methylcyclohexan bzw. Toluol.

Im Rahmen der Erfindung können Phasentransferkatalysatoren nach Maßgabe des Fachmanns benutzt werden. Geeignete sind in DE19752041 aufgeführt. Besonders bevorzugt ist ein quartäres Ammoniumsalz wie Tetrabutylammoniumbromid bzw. - chlorid. Üblicherweise werden nach dem erfindungsgemäßen Verfahren 0,1 bis 5 Mol-% Katalysator bezogen auf die eingesetzte C-H-acide Verbindung genommen. Vorzugsweise kommt der Katalysator in 0,25 bis 1 Mol-%, besonders bevorzugt um 0,5 Mol-%, zum Einsatz, wobei die Zugabe sowohl portionsweise als Feststoff oder vorzugsweise gelöst in einem Lösungsmittel oder dem eingesetzten Dihalogenalkan erfolgen kann.

Es hat sich als vorteilhaft erwiesen, wenn das methylengruppentragende Substrat, das Dihalogenalkan und das Carbonat im Molverhältnis 1:2,0-5,0:1,0-2,0 eingesetzt werden. Bevorzugt ist jedoch der Einsatz in einem Molverhältnis von 1:2,5-3,5:1,2-1,8. Das überschüssige Alkylhalogenid kann nach der Reaktion zurückgewonnen und erneut in die Reaktion eingeschleust werden. Überraschend und unversehrsehbar im Sinne der Erfindung ist dabei, dass trotz eingesetzter Überschüsse an Dihalogenalkan und Carbonat nur in sehr geringem Umfang bisalkylierte Produkte mit der erfindungsgemäßen Verfahrensweise erzeugt werden.

Die gegenständliche Reaktion wird vorteilhaft in einem polar aprotischen Lösungsmittel durchgeführt. Alternativ kann auch ein ein- oder zweifach endgruppenverschlossener Polyether wie Polyethylenglykol als Lösungsmittel herangezogen werden (DE19963115). Auch Gemische beider Lösungsmittelgruppen können als Reaktionsmedium dienen. Wird der Polyether im Gemisch mit dem polar aprotischen Lösungsmittel eingesetzt, ist es ausreichend, diesen in 1-20 Gew.-%, vorzugsweise 5-15 Gew.-% bezogen auf das polar aprotische Lösungsmittel zu dosieren. Als Alternative zu Polyethern können auch Kronenether genommen werden. Durch Zugabe dieser Reagenzien wird eine bessere Löslichkeit der Base im organischen Lösungsmittel erreicht.
Als aprotisch polare Lösungsmittel kommen alle dem Fachmann für diesen Zweck zur Verfügung stehenden unter den gegebenen Reaktionsbedingungen indifferenten in Frage. Vorzugsweise sind dies DMF, DMSO, Dimethylacetamid oder N-Methylpyrrolidon.

Die gegenständliche Erfindung wird vorteilhaft so ausgeführt, dass man Lösungsmittel, Dihalogenalkan und Carbonat-Base mit Schleppmittel vorlegt und das Gemisch zum Sieden erhitzt. Anschließend dosiert man Phasentransferkatalysator und die C-H-acide methylengruppentragende Verbindung zusammen in einer solchen Geschwindigkeit ein, dass Gasentwicklung und Reaktionsspitzen unter Kontrolle gehalten werden können. In der Regel ist die Reaktion innerhalb von 3 bis 4 Stunden abgeschlossen. Die Zugabe des Phasentransferkatalysators kann jedoch auch getrennt von der Zugabe der C-H-aciden methylengruppentragenden Verbindung erfolgen.
In Vergleichsexperimenten hat sich gezeigt, dass bei vollständiger Katalysatorzugabe zu Beginn der Reaktion nach kurzer Zeit eine unvorhersehbare Abnahme der Katalysatoraktivität beobachtet wird, die zu unvollständigen Umsätzen und schlechten Ausbeuten führt. Der Katalysator wird daher kontinuierlich ab dem Beginn der Reaktion zur Reaktionsmischung zugegeben. Dies kann über den gesamten Zeitraum der Reaktion geschehen, oder auch nur innerhalb des ersten Drittels bis zur Hälfte des Reaktionszeitraums. Wie schon angedeutet hat es sich als besonders vorteilhaft erwiesen, die C-H-acide Komponente ebenfalls zuzudosieren, da so insbesondere die Gasentwicklung in einem technisch gut beherrschbaren Rahmen gehalten werden kann. Es wurde schon darauf hingewiesen, dass der Reaktionsverlauf vorteilhaft anhand der gebildeten Gas- und Wassermenge verfolgt werden kann.

Die Aufarbeitung der Reaktionsmischung erfolgt üblicherweise durch einfache mechanische Abtrennung des Salzes und anschließende Destillation des Filtrats. Sowohl das eingesetzte Schleppmittel als auch das überschüssige Dihalogenalkan können bei der Destillation zurückgewonnen und erneut in die Reaktion eingesetzt werden. Der Anteil gebildeter bisalkylierter Nebenprodukte liegt üblicherweise bei <5 Gew.-%.

Gegenstand der Erfindung ist ebenfalls ein Verfahren zur Herstellung von ω-Halogenalkylnitrilen bzw. -carbonsäuren. Bei diesem werden Malonsäurediester bzw. Cyanessigsäureester mit eingangs erwähnten α, ω-Dihalogenalkanen gemäß den eben dargelegten Maßnahmen umgesetzt und diese anschließend verseift und decarboxyliert.
In einer bevorzugten Variante des Verfahrens kann das nach Abtrennung des Schleppmittels und des überschüssigen Alkylhalogenids erhaltene Rohprodukt aus der weiter oben beschriebenen Monoalkylierungsreaktion direkt in einem nachfolgenden Verseifungs-/Decarboxylierungsschritt zur Herstellung der entsprechenden Monosäure/-nitrile eingesetzt werden. Auch eine Destillation des Rohprodukts an einem Kurzweg- oder Dünnschichtverdampfer ist möglich. Diese Variante ist vor allem für langkettige Alkylierungsprodukte (n ∼ 8) vorteilhaft, da in diesen Fällen eine Destillation aufgrund der hohen Siedepunkte technisch nur schwer durchführbar ist.

Vorzugsweise führt man die Verseifung und Decarboxylierung durch Zusetzen einer Katalysatorsäure ohne Zusatz eines weiteren Lösungsmittels durch.
Die Herstellung der entsprechenden Monocarbonsäurederivate wie z.B. ω-Halogencarbonsäureester und ω-Halogennitrile erfolgt dabei vorteilhafterweise dadurch, dass die durch Monoalkylierung hergestellten Derivate ohne Zusatz von Lösemittel mit einer geringen Menge Katalysatorsäure versetzt, auf Reaktionstemperatur gebracht und dann mit Wasser bzw. einer kurzkettigen Carbonsäure umgesetzt werden. Auf diese Weise bilden sich durch Hydrolyse bzw.

Acidolyse und nach spontaner Decarboxylierung die gewünschten aliphatischen ω-Halogencarbonsäureester bzw. ω-Halogenalkylnitrile in nahezu quantitativer Ausbeute. Sofern es sich bei dem Produkt um ein Carbonsäureester handelt, kann es dazu kommen, dass dieser unter den Reaktionsbedingungen bis zur Carbonsäure umgesetzt wird. In diesem Fall kann nach dem erfindungsgemäßen Verfahren durch Zugabe von Alkohol eine Rückveresterung erfolgen.

In einer besonders geeigneten Verfahrensdurchführung werden zur Decarboxylierung die Monoalkylierungsprodukte und die Katalysatorsäure ohne Lösemittel vorgelegt, auf Reaktionstemperatur erhitzt und anschließend Wasser bzw. eine kurzkettige Carbonsäure kontinuierlich zugefördert. Auf diese Weise werden die während der Reaktion entstehenden Leichtsieder destillativ über Kopf abgetrennt. Der Reaktionsverlauf kann vorteilhaft anhand der gebildeten Kohlendioxidmenge verfolgt werden.

Als Katalysatorsäure können zur Hydrolyse bzw. Acidolyse anorganische Säuren eingesetzt werden. Besonders geeignet sind je nach Siedepunkt des Produktes Sulfonsäuren wie Methansulfonsäure, *para*- Toluolsulfonsäure oder längerkettige Alkylbenzolsulfonsäuren. Üblicherweise werden nach dem erfindungsgemäßen Verfahren 0,5-10 Gew-% Katalysatorsäure bezogen auf die eingesetzten Derivate wie Malon- oder Cyanessigester eingesetzt. In einer bevorzugten Durchführung werden lediglich 2 bis 5 Gew-% Katalysator zugesetzt.

Sowohl die Acidolyse bzw. Hydrolyse als auch die optionale Rückveresterung werden vorteilhaft in einem Temperaturbereich von 100-200°C, bevorzugt im Temperaturbereich von 120-150°C durchgeführt.

Im Fall der substituierten Malonsäurealkylester erfolgt nach vollständiger Hydrolyse eine Nachveresterung der zum Teil vorliegenden Carbonsäure mit dem entsprechenden Alkohol. In einer bevorzugten Variante wird dabei der Alkohol direkt in den auf Reaktionstemperatur gebrachten Sumpf gefördert und freiwerdendes Wasser destillativ über Kopf abgetrennt

Die Reinigung der Carbonsäureester bzw. Alkylnitrile kann entweder an einer Destillationskolonne oder einem Kurzweg- bzw. Dünnschichtverdampfer erfolgen. Die zurückbleibende hochsiedende Katalysatorsäure kann erneut in die Reaktion eingesetzt werden

Die gewünschten Carbonsäureester und Alkylnitrile werden nach dieser Variante in sehr hoher Reinheit und mit Ausbeuten von bis zu 95% d. Th. erhalten. Als bevorzugt herzustellende Verbindungen gelten in diesem Zusammenhang: 8-Chloroctansäureethylester, 10-Chlordecansäureethylester sowie 8-Bromoctansäureethylester.

Als Alkyl/Alkan wird vorzugsweise ein linearer oder verzweigtes (C₁-C₁₅)-Alkyl/Alkan, insbesondere ein (C₁-C₈)-Alkyl/Alkan verstanden, wie z.B. Methyl, Ethyl, Propyl, Isopropyl, Butyl, tert.-Butyl, sec-Butyl, Isobutyl, Pentyl, Hexyl, Dodecyl- etc. Ein (C₁-C₈)-Alkyl/Alkan ist entsprechend jedoch mit maximal 8 C-Atomen zu sehen.

Unter Halogen versteht man im Rahmen der Erfindung ein Fluor, Chlor, Brom oder Jod.

Die folgenden Beispiele sollen das erfindungsgemäße Verfahren näher erläutern, nicht jedoch auf die speziell genannten Umstände einschränken.

### Beispiele:

### 1. Alkylierung von Malonsäurediethylester mit 1,6-Dichlorhexan:

In einem 21-Reaktionsgefäß mit 50 cm-Kolonne, Kühler und Wasserauskreiser werden 400 g (3,0 mol) 1,6-Dichlorhexan, 207 g (1,5 mol) Kaliumcarbonat, 200 ml DMF und 300 ml Cyclohexan bei Raumtemperatur vorgelegt. Der Wasserauskreiser wird zusätzlich mit 45 ml Cyclohexan befüllt. Die Reaktionsmischung wird unter Rühren zum Sieden erhitzt, so dass das am Kolonnenkopf kondensierte Destillat in den Wasserauskreiser und anschließend direkt zurück in den Reaktionssumpf gefahren wird. Anschließend werden gleichzeitig 160 g Diethylmalonat (1,0 mol) und eine Lösung von 1,6 g Tetrabutylammoniumbromid in 65 g 1,6-Dichlorhexan innerhalb von 1 h zudosiert. Der Reaktionsverlauf kann anhand der entwickelten Gasmenge sowie der gebildeten Wassermenge verfolgt werden. Nach weiteren 2 h ist die Reaktion beendet (Umsatz DEM >99%) und die Reaktionsmischung wird abgekühlt, filtriert und mit Cyclohexan gewaschen. Das Filtrat wird anschließend im Vakuum destilliert. Dabei werden 206 g (0,74 mol) 6-Chlorhexylmalonsäurediethylester erhalten (Ausbeute: 74 %, Reinheit >99 %)

### 2. Alkylierung von Malonsäurediethylester mit 1,6-Dichlorhexan:

In der in Beispiel 1 beschriebenen Apparatur werden 400 g (3,0 mol) 1,6-Dichlorhexan, 207 g (1,5 mol) Pottasche, 6,3 g Polyethylenglycolmonomethylether (M500) und 345 ml Cyclohexan bei Raumtemperatur vorgelegt und unter Rühren zum Sieden erhitzt. Anschließend werden gleichzeitig 160 g DEM (1,0 mol) und eine Lösung von 1,6 g Tetrabutylammoniumbromid in 65 g 1,6-Dichlorhexan innerhalb einer Stunde zudosiert. Nach weiteren 2 h Nachreaktionszeit (Umsatz DEM >99%) wird die Reaktionsmischung abgekühlt, filtriert, mit Cyclohexan nachgewaschen und das Filtrat im Vakuum destilliert. Es werden 218 g (0,78 mol) 6-Chlorhexylmalonsäurediethylester erhalten (Reinheit >99 %, Ausbeute: 78 %).

### 3. Alkylierung von Malonsäurediethylester mit 1,8-Dichloroctan:

Analog dem in Beispiel 1 beschriebenen Verfahren werden 659 g (3,6 mol) 1,8-Dichloroctan und 248 g (1,75 mol) Kaliumcarbonat mit 192 g DEM in Gegenwart von 2 g Tetrabutylammoniumbromid, 7,6 g Polyethylenglycolmonomethylether (M500), 400 ml Cyclohexan und 50 ml DMF umgesetzt. Bei vollständigem DEM-Umsatz (>99 %, ca. 3 h) wird die Reaktion beendet und die Mischung wie in Beispiel 1 beschrieben aufgearbeitet. Es werden 283 g 8-Chloroctylmalonsäurediethylester erhalten (Reinheit >99 %, Ausbeute: 77 %).

### 4. Alkylierung von Malonsäurediethylester mit 1,6-Dibromhexan:

Analog dem in Beipiel 1 beschriebenen Verfahren werden 2045 g (8,38 mol) 1,6-Dibromhexan und 579 g (4,19 mol) Kaliumcarbonat mit 448 g (2,79 mol) Diethylmalonat in Gegenwart von 17,6 g Polyethylenglycolmonomethylether (M500), 4,5 g Tetrabutyl-ammoniumbromid und 950 ml Cyclohexan umgesetzt. Es werden 701 g 6-Bromhexylmalonsäurediethylester erhalten (Reinheit >99 %, Ausbeute: 78 %) .

### 5. Vergleichsbeispiel: Pottasche-Alkylierung mit vorgelegtem Katalysator:

Eine Mischung aus 622 g Kaliumcarbonat (4,5 mol), 18,9 g Polyethylenglycol-monomethylether (M500), 1396 g (9,0 mol) 1,6-Dichlorhexan, 4,8 g Tetrabutylammonium-bromid und 950 ml Cyclohexan wird vorgelegt und am Wasserauskreiser zum Sieden erhitzt. In die Reaktionsmischung werden innerhalb von 2 h 481 g (3,0 mol) Diethylmalonat dosiert. Nach weiteren 4 h Reaktionszeit beträgt der DEM-Umsatz lediglich 59 %. Nach Aufarbeitung der Reaktionsmischung und Destillation werden 308 g 6-Chlorhexylmalonsäurediethylester erhalten (Ausbeute: 37 %)

### 6. Vergleichsbeispiel: Alkylierung von Malonsäurediethylester mit 1,6-Dichlorhexan unter Einsatz von Natriumethanolat als Base:

Gemäß DE 3401913 werden zu einer Lösung von 10 mol Natriumethylat in 4,5 1 trockenem Ethanol bei 70 °C 10 mol Malonsäuredietyhlester zugetopft und so das Natriumsalz des Malonsäurediethylesters gebildet. Die Lösung wird noch warm in eine unter Rückfluss kochende Mischung aus 5 1 trockenem Ethanol und 20 mol 1,6-Dichlorhexan eingerührt. Nach erfolgter Reaktion wird das Ethanol im Vakuum entfernt und der Rückstand mit Chloroform aufgenommen. Nach Absaugen der entstandenen Salze und Entfernen des Chloroforms im Vakuum wird das Produkt im Vakuum fraktioniert destilliert (Ausbeute 65 %). Bei dieser Reaktionsführung konnten bis zu 500 g ungewünschte bisalkylierte Nebenprodukte, insbesondere 2,2-Bis-(6-chlorhexyl)-malonsäurediethylester und 2,9-Bis-ethoxycarbonyldecandisäurediethylester, isoliert werden.

### 7. Umsetzung von 2-(6-Chlorhexyl)-malonsäurediethylester zu 8-Chloroctansäure-ethylester

In einem 500 ml-Reaktionsgefäß mit Kühler und Kolonne werden 1 mol 2-(6-Chlorhexyl)-malonsäurediethylester und 5 Gew-% Marlon® AS3-Säure bei 150 °C vorgelegt. Über einen Zeitraum von 3 h werden 3 mol Wasser zudosiert und das entstehende Ethanol als Gemisch mit Wasser über Kopf abdestilliert. Nach vollständigem Umsatz des Eduktes werden 3 mol Ethanol innerhalb von 3 h in den heißen Sumpf geleitet, Reaktionswasser über Kopf abgetrennt und die teilweise entstandene 8-Chloroctansäure wieder verestert. Nach Destillation des Rückstandes im Vakuum erhält man den gewünschten 8-Chloroctansäureethylester mit einer Reinheit von >99 % und in einer Ausbeute von 95 % d. Th..

### 8. Umsetzung von 2-(8-Chloroctyl)-malonsäurediethylester zu 10-Chlordecansäure-ethylester

Analog zu dem in Beispiel 7 beschriebenen Verfahren werden 1 mol 2-(8-Chloroctyl)-malonsäurediethylester mit 5 Gew% Marlon® AS3-Säure bei 150 °C vorgelegt. Über einen Zeitraum von 3 h werden 3 mol Wasser zudosiert und freiwerdenes Ethanol als Gemisch mit Wasser über Kopf abdestilliert. Nach vollständigem Umsatz des Eduktes werden 3 mol Ethanol innerhalb von 3 h in den heißen Sumpf geleitet, das entstehende Reaktionswasser über Kopf abgetrennt und die bereits gebildete 10-Chloroctansäure wieder verestert. Nach Destillation des Rückstandes im Vakuum erhält man den 10-Chlordecansäureethylester mit einer Reinheit von >99 % und in einer Ausbeute von 90 % d. Th..

### 9. Umsetzung von 2-(6-Bromhexyl)-malonsäurediethylester zu 8-Bromoctansäure-ethylester

Analog zu dem in Beispiel 7 beschriebenen Verfahren werden 1 mol 2-(8-Bromhexyl)-malonsäurediethylester mit 5 Gew% Marlon® AS3-Säure bei 150 °C vorgelegt. Über einen Zeitraum von 3-4 h werden 4 mol Wasser zudosiert und freiwerdenes Ethanol als Gemisch mit Wasser über Kopf abdestilliert. Nach vollständigem Umsatz des Eduktes werden 4 mol Ethanol innerhalb von 3-4 h in den heißen Sumpf geleitet, Reaktionswasser über Kopf abgetrennt und die teilweise entstandene 8-Bromoctansäure wieder verestert. Nach Destillation des Rückstandes im Vakuum erhält man den 8-Bromoctansäureethylester mit einer Reinheit von >99 % und in einer Ausbeute von 90 % d. Th.

### 10. Alkylierung von Malonsäurediethylester mit 1,6-Dichlorhexan

Analog dem in Beispiel 1 beschriebenen Verfahren wurde eine Suspension aus 622 g Kaliumcarbonat, 1200 g 1,6-Dichlorhexan, 18,9 g Polyglycol M500 und 800 ml Cyclohexan zum Sieden erhitzt und zunächst innerhalb einer Stunde 481 g Diethylmalonat und nach Abschluß der DEM-Zugabe innerhalb einer Stunde eine Lösung von 4,8 g TBAB in 195 g Dichlorhexan zugetropft. Der Reaktionsverlauf konnte anhand der Gasentwicklung verfolgt werden .

| Reaktionszeit: | Gasmenge: | Umsatz: |
|---|---|---|
| 1 h | 0,2 l | 0,6% |
| 2 h | 2,6 l | 7% |
| 4 h | 17,8 l | 51% |
| 6 h | 35,1 l | 100% |

Aufarbeitung und Reinigung analog Beispiel 1 ergab 625 g 6-Chlorhexylmalonsäurediethylester (75% Ausbeute).

## Patentansprüche

1. Verfahren zur Monalkylierung C-H-acider Methylengruppen durch Reaktion des methylengruppentragenden Substrats in einem polaren aprotischen Lösungsmittel mit Dihalogenalkanen, bei denen die beiden Halogenatome durch eine Kette von mindestens 3 C-Atomen getrennt sind, in Gegenwart von Alkali- oder Erdalkalicarbonaten und einem Phasentransferkatalysator unter stetiger Entfernung des sich bildenden Reaktionswassers,
**dadurch gekennzeichnet, dass**
man die Alkali- oder Erdalkalicarbonate im Verhältnis zum methylengruppentragenden Substrat von >0.6:1 einsetzt.

2. Verfahren nach Anspruch 1,
**dadurch gekennzeichnet, dass**
man die Alkali- oder Erdalkalicarbonate im Verhältnis zum methylengruppentragenden Substrat von >1:1 einsetzt.

3. Verfahren nach Anspruch 1 und/oder 2,
**dadurch gekennzeichnet, dass**
man den Phasentransferkatalysator kontinuierlich ab Beginn der Alkylierungsreaktion zur Reaktionsmischung zudosiert.

4. Verfahren nach Anspruch 1, 2 und/oder 3,
**dadurch gekennzeichnet, dass**
man methylengruppentragende Substrate ausgewählt aus der Gruppe der 1,3-Dicarbonylverbindungen benutzt.

5. Verfahren nach einem oder mehreren der vorhergehenden Ansprüche,
**dadurch gekennzeichnet, dass**
man Kaliumcarbonat einsetzt.

6. Verfahren nach einem oder mehreren der vorhergehenden Ansprüche,
**dadurch gekennzeichnet, dass**
man als zur Entfernung des Reaktionswassers ein Schleppmittel ausgewählt aus der Gruppe der cyclischen Kohlenwasserstoffe oder aromatischen Kohlenwasserstoffe einsetzt.

7. Verfahren nach einem oder mehreren der vorhergehenden Ansprüche,
**dadurch gekennzeichnet, dass**
man als Phasentransferkatalysator ein quartäres Ammoniumsalz einsetzt.

8. Verfahren nach einem oder mehreren der vorhergehenden Ansprüche,
**dadurch gekennzeichnet, dass**
man das methylengruppentragende Substrat, das Alkylhalogenid und das Carbonat im Verhältnis 1:2,0-5,0:1,0-2,0 einsetzt.

9. Verfahren nach einem oder mehreren der vorhergehenden Ansprüche,
**dadurch gekennzeichnet, dass**
als polar aprotisches Lösungsmittel DMF, DMSO, Dimethylacetamid oder N-Methylpyrrolidon eingesetzt wird.

10. Verfahren zur Herstellung von ω-Halogenalkylnitrilen bzw. -carbonsäuren,
**dadurch gekennzeichnet, dass**
man Malonsäurediester bzw. Cyanessigsäureester mit α, ω- Dihalogenalkanen nach Anspruch 1 umsetzt und diese anschließend verseift und decarboxyliert.

11. Verfahren nach Anspruch 10,
**dadurch gekennzeichnet, dass**
man die Verseifung und Decarboxylierung durch Zusatz einer Katalysatorsäure ohne Zusatz eines Lösungsmittels durchführt.
